(19)
Europäisches
Patentamt
European
Patent Office
Office européen
des brevets

(11) **EP 3 061 481 B1**

(12) **EUROPÄISCHE PATENTSCHRIFT**

(45) Veröffentlichungstag und Bekanntmachung des Hinweises auf die Patenterteilung:
**10.01.2018 Patentblatt 2018/02**

(21) Anmeldenummer: **16000466.9**

(22) Anmeldetag: **26.02.2016**

(51) Int Cl.:
***A61M 16/00*** *(2006.01)*

(54) **VORRICHTUNG ZUR VOLUMENBHÄNGIGEN TRIGGERUNG DER AUSATEMPHASE**

DEVICE FOR VOLUME-DEPENDENT TRIGGERING OF THE EXHALATION PHASE

DISPOSITIF DE DÉCLENCHEMENT DE PHASE EXPIRATOIRE EN FONCTION DU VOLUME

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.02.2015 DE 102015002491**

(43) Veröffentlichungstag der Anmeldung:
**31.08.2016 Patentblatt 2016/35**

(73) Patentinhaber: **Löwenstein Medical Technology S.A.**
**2557 Luxembourg (LU)**

(72) Erfinder:
- **EISENHUBER, Mischa**
  **24558 Henstedt-Ulzburg (DE)**
- **PELIKAN, Jerns**
  **23558 Lübeck (DE)**

(74) Vertreter: **Marx, Thomas**
**Löwenstein Medical Technology GmbH + Co. KG**
**IP Management**
**Kronsaalsweg 40**
**22525 Hamburg (DE)**

(56) Entgegenhaltungen:
**WO-A1-2005/075013**
**WO-A1-2013/182944**
**WO-A1-2014/199264**
**US-A1- 2014 373 845**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Unter Beatmung wird die teil- oder vollständige Übernahme der physiologischen Atmung durch ein Beatmungsgerät verstanden. Unterschieden wird zwischen verschiedenen Beatmungsformen. Bei der assistierten Beatmung unterstützt das Beatmungsgerät den Patienten in seiner Eigenatmung und folgt dabei dem Atemrhythmus des Patienten. Dies kann auch nur bedarfsweise erfolgen. Bei der kontrollierten Beatmung wird die zeitliche Steuerung durch das Beatmungsgerät vorgegeben; die Beatmung dient dann im Idealfall der vollständigen Entlastung der Atemmuskulatur (Atempumpe). Assistierte wie kontrollierte Beatmung können im klinischen wie im außerklinischen Umfeld (zu Hause, auf einer Pflegestation) erfolgen.
Um die Beatmung zu parametrisieren, werden abhängig von der konkreten Beatmungsform Regelgrößen für Fluss, Volumen, Druck und/oder Zeit vorgegeben. Eine oder mehrere dieser Größen werden Kontrollvariablen genannt und während jedes Atemzugs geregelt. Verbreitet sind Beatmungsformen mit einer Volumen- oder Druckvorgabe. Andere Größen ergeben sich unter der Beatmung abhängig von der Kontrollvariablen und Größen der Lungenmechanik (Atemwegimpedanz: R, C) und auch vom verwendeten Zubehör. Die Compliance (C) und Resistance (R) des Patienten wirken also mit auf die jeweils nicht vorgegebene(n) Größe(n).

Die WO 2005/075013 A1 beschreibt ein Beatmungsgerät mit einem Druckgenerator zur Förderung von Atemgas unter Inspirationsdruck und Exspirationsdruck. Das Ziel der WO 2005/075013 A1 ist die Titration des patientenindividuell (richtigen) Inspirationsdrucks, um eine möglichst große Entlastung des Patienten zu erreichen. Dazu wird der Inspirationsdruck von Beatmungszyklus zu Beatmungszyklus stufenförmig angehoben. Als Analysesignal wird das sich passiv einstellende Atemgas-Volumen bestimmt. Ein Anzeichen für die Entlastung liegt dann vor, wenn nach einer Druckerhöhung (für den Inspirationsdruck) das Beatmungsvolumen abnimmt.
Die US 2014/0373845 A1 offenbart ebenfalls ein Beatmungsgerät mit einem Druckgenerator, wobei ebenfalls der patientenindividuell (richtige) Inspirationsdruck gefunden werden soll. Dazu werden für einen vorgegebenen Druck unterschiedliche Beatmungsparameter und die Atemarbeit des Patienten analysiert.
Die WO 2013/182944 A1 offenbart ein Gerät zur Unterstützung des Abhustens unter der Beatmung.
Die WO 2014/199264 A1 offenbart ein Beatmungsgerät mit einem Druckgenerator, wobei die Patientenatmung analysiert wird und der inspiratorische und der exspiratorische Fluss des Patienten überwacht werden, um synchron zu der Patientenatmung eine Druckunterstützung zu applizieren.

An grundsätzlichen Beatmungsformen wird unterschieden: Bei Beatmung mit Druckvorgabe kann der Atemgasfluss meist nicht vom Beatmungsgerät vorgegeben werden, und das Beatmungsvolumen ergibt sich im Wesentlichen aus den lungenmechanischen Größen des Patienten sowie abhängig vom technischen Equipment (z.B. verwendeter Beatmungsschlauch und Patienten-Interface).

Bei Beatmung mit Volumenvorgabe (Volume controlled ventilation, VCV) wird innerhalb des Atemzugs der Fluss das über eine ebenfalls vorgegebene Zeit geregelt. Bei dieser Beatmungsform ist mit den gewählten Größen Volumen und Zeit auch ein Flussprofil vorgegeben. Der applizierte Druck ergibt sich abhängig von diesem Flussprofil, der Lungenmechanik und dem Zubehör. Dabei kann der Druck, zumindest in Form von Druckspitzen, auch so hoch sein, dass die Gefahr einer Lungenschädigung besteht. Um dies zu vermeiden, kann auch bei Beatmung mit Volumenvorgabe dieses Zielvolumen mit einem maximalen Druck im Sinne einer Alarmgrenze gekoppelt werden, wobei jedoch gemäß dem Stand der Technik immer eine feste Inspirationszeit vorgegeben ist.

Bei der Beatmung mit Druckvorgabe erfolgt die Umschaltung von der Inspiration in die Exspiration optional über einen Patienten-Trigger. Hierbei wird das Flow-Signal als Trigger berücksichtigt. Beispielsweise erfolgt eine Umschaltung in die Exspirationsphase nach Unterschreitung einer Triggerschwelle, die das Ende der Lungenfüllung erkennen lässt.

Es besteht ein Bedarf dem Patienten in jeder Inspiration ein vorbestimmtes Volumen an Atemgas ohne beschriebene Nachteile der VCV Beatmung zu verabreichen, und ohne die Lunge schädigenden Einflüsse durch hohen Druck auszusetzen.

Erfindungsgemäß wird dazu ein Beatmungsgerät vorgeschlagen mit einem Druckgenerator zur Förderung von Atemgas unter Inspirationsdruck und Exspirationsdruck zu einem Patienten Interface, zumindest einem Flow- und/oder Drucksensor, einer Eingabeeinheit zur Vorgabe zumindest eines Zielvolumens und/oder eines Druckwertes für eine Inspirationsphase sowie zumindest einem Speicher zur abrufbaren Speicherung von Zielvolumen und von Druckwerten wobei der Druckgenerator während der Inspirationsphase das Atemgas unter Druck fördert und ein Volumensteuerungs-Modul das geförderte Atemgasvolumen ermittelt und mit dem Zielvolumen vergleicht und es den Druckgenerator in Abhängigkeit von der Erreichung des Zielvolumens zur Umschaltung in eine Exspirationsphase veranlasst.

Erfindungsgemäß wird auch ein Beatmungsgerät vorgeschlagen mit einem Druckgenerator zur Förderung von Atemgas unter Inspirationsdruck und Exspirationsdruck zu einem Patienten Interface mit zumindest einem Flow- und/oder Drucksensor einer Eingabeeinheit zumindest zur Vorgabe eines Zielvolumens für eine Inspirationsphase sowie zumindest einem Speicher zur abrufbaren Speicherung von Zielvolumen und von Druckwerten und einer Steuereinheit, die zumindest ein Steuermodul aufweist, wobei der Druckgenerator während der Inspirationsphase das Atemgas unter Druck fördert und das Volumensteuerungs-Modul der Steuereinheit das geförderte Atemgasvolumen ermittelt und mit dem Zielvolumen vergleicht und es den Druckgenerator in Abhängigkeit von der Erreichung des Zielvolumens zur Umschaltung in eine Exspirationsphase veranlasst.

Das erfindungsgemäße Beatmungsgerät kennzeichnet sich auch dadurch, dass das Volumensteuerungs-Modul ein Akzeptanzfenster für das Zielvolumen bereitstellt.

Das erfindungsgemäße Beatmungsgerät kennzeichnet sich auch dadurch, dass das Akzeptanzfenster eine Abweichung von bis zu 20 Prozent vom Zielvolumen umfasst.

Das erfindungsgemäße Beatmungsgerät kennzeichnet sich auch dadurch, dass das Volumensteuerungs-Modul Daten des Flusssensors und/oder Drucksensors erhält und daraus das in der Inspirationsphase geförderte Atemgasvolumen ermittelt, wobei das Volumensteuerungs-Modul eine Schnittstelle aufweist über die Daten mit anderen Modulen ausgetauscht werden und wobei das Volumensteuerungs-Modul das in der Inspirationsphase geförderte Atemgasvolumen mit dem im Speicher hinterlegten Zielvolumen vergleicht und in Abhängigkeit von der Erreichung des Zielvolumens ein Triggersignal generiert, welches den Druckgenerator direkt oder indirekt (über das Drucksteuerungsmodul) zur Umschaltung in eine Exspirationsphase veranlasst.

Das erfindungsgemäße Beatmungsgerät kennzeichnet sich auch dadurch, dass die Steuereinheit ein Drucksteuerungsmodul aufweist, welches den Druckgenerator ansteuert und welches eine Schnittstelle aufweist, über die Daten mit anderen Modulen ausgetauscht werden, wobei das Drucksteuerungsmodul den Druckgenerator zumindest in der Inspirationsphase entsprechend der im Speicher hinterlegten Druckwerte und/oder dem im Speicher hinterlegten Zieldruck und/oder dem im Speicher hinterlegten Druckprofil angesteuert.

Das erfindungsgemäße Beatmungsgerät kennzeichnet sich auch dadurch, dass die Steuereinheit ein Zeitkontrollmodul aufweist, welches in dem Speicher einen Zeitstempel des Inspirationsbeginns und eine Zeiterfassung der Inspiration aufzeichnet, wobei das Zeitkontrollmodul über seine Schnittstelle, über die Daten mit anderen Modulen ausgetauscht werden, auf die Status Information, in welcher Phase sich die Ventilation befindet, zugreift und wobei das Zeitkontrollmodul die in der Inspiration verstrichene Zeit erfasst und diese anderen Modulen über seine Schnittstelle bereitstellt.

Das erfindungsgemäße Beatmungsgerät kennzeichnet sich auch dadurch, dass die Steuereinheit ein Triggermodulaufweist, welches ermittelt, ob der exspiratorische Trigger ausgelöst werden kann, indem ermittelt wird, ob das Zielvolumen erreicht wurde oder ob die maximale Inspirationszeit erreicht wurde, wobei das Triggermodul über seine Schnittstelle das verabreichte Volumen an Atemgas abgreift und dieses mit dem gespeicherten Zielvolumen vergleicht und wobei das Triggermodul über seine Schnittstelle die Zeit seit Inspirationsbeginn abgreift und diese mit der gespeicherten maximalen Inspirationszeit vergleicht und wobei das Triggermodul den exspiratorischen Trigger ausgelöst, wenn das Zielvolumen erreicht ist und/oder die maximalen Inspirationszeit erreicht ist.

Das erfindungsgemäße Beatmungsgerät kennzeichnet sich auch dadurch, dass die Steuereinheit ein Zeitkontroll-Modul aufweist, welches den zeitlichen Verlauf der Inspiration überwacht und aufzeichnet, wobei das Zeitkontroll-Modul die Inspiration beendet wenn die maximale Inspirationszeit Ti,max abgelaufen ist, woraufhin das Drucksteuerungsmodul mit einem Druckübergang auf einen exspiratorischen Druck beginnt.

Das erfindungsgemäße Beatmungsgerät kennzeichnet sich auch dadurch, dass die Steuereinheit ein Alarmmodul aufweist, welches mit dem Ende der Inspiration ein akustisches Alarmsignal generiert, wenn das Zielvolumen nicht im Rahmen des Akzeptanzfensters erreicht wird.

Das erfindungsgemäße Beatmungsgerät kennzeichnet sich auch dadurch, dass der Druckgenerator während der Inspirationsphase das Atemgas unter dem vorgegebenen Druck oder entsprechend dem vorgegebenen Druckprofil fördert.

Das erfindungsgemäße Beatmungsgerät kennzeichnet sich auch dadurch, dass der Druckgenerator alternative Druckanstiegsrampen als Drucksollkurve vorgibt und/oder ausgewählt aus Rampen mit verschiedener Steigung oder linear oder stetig steigend oder rampenförmig oder mit weichen Übergängen.

Das erfindungsgemäße Beatmungsgerät kennzeichnet sich auch dadurch, dass über die Eingabeeinheit die Vorgabe eines Zielvolumens und zumindest eines Druckwertes oder einer Druckanstiegsrampe für eine Inspirationsphase erfolgt.

Das erfindungsgemäße Beatmungsgerät kennzeichnet sich auch dadurch, dass von der Inspiration in die Exspiration geschaltet wird, wenn nach Ablauf einer definierbaren Zeitspanne das Zielvolumen nicht erreicht wurde.

Das erfindungsgemäße Beatmungsgerät kennzeichnet sich auch dadurch, dass nach Ablauf der maximalen Inspirationszeit ein akustischer und/oder optischer Alarm ausgelöst wird.

Erfindungsgemäß wird auch Verfahren zur Steuerung eines Beatmungsgerätes vorgeschlagen mit einem Druckgenerator zur Förderung von Atemgas unter Inspirationsdruck und Exspirationsdruck zu einem Patienten Interface, mit zumindest einem Flow- und/oder Drucksensor, einer Eingabeeinheit zumindest zur Vorgabe eines Zielvolumens für eine Inspirationsphase sowie zumindest einem Speicher zur abrufbaren Speicherung von Zielvolumen und von Druckwerten und einer Steuereinheit, die zumindest ein Steuermodul aufweist, wobei der Druckgenerator während der Inspirationsphase das Atemgas unter Druck fördert und das Volumensteuerungs-Modul der Steuereinheit das geförderte Atemgasvolumen ermittelt und mit dem Zielvolumen vergleicht und es den Druckgenerator in Abhängigkeit von der Erreichung des Zielvolumens zur Umschaltung in eine Exspirationsphase veranlasst.

Das erfindungsgemäße Beatmungsgerät kennzeichnet sich auch dadurch, dass das Volumensteuerungs-Modul ein Akzeptanzfenster für das Zielvolumen bereitstellt.

Das erfindungsgemäße Beatmungsgerät kennzeichnet sich auch dadurch, dass das Akzeptanzfenster eine Abweichung von bis zu +/-20 Prozent vom Zielvolumen umfasst.

Das erfindungsgemäße Beatmungsgerät kennzeichnet sich auch dadurch, dass der Druckgenerator während der Inspirationsphase das Atemgas unter dem vorgegebenen Druck oder entsprechend dem vorgegebenen Druckprofil fördert. Mit Beginn der Inspiration wird der Beatmungsdruck entlang einer Rampenfunktion (Druckanstiegsrampe) erhöht. Das vorgegebene Volumen wird permanent überwacht. Ist das angegebene Volumen gleich dem Zielvolumen, so wird die Inspiration beendet. Auch wenn der eingestellte maximale inspiratorische Druck (IPAP) noch nicht erreicht wurde.

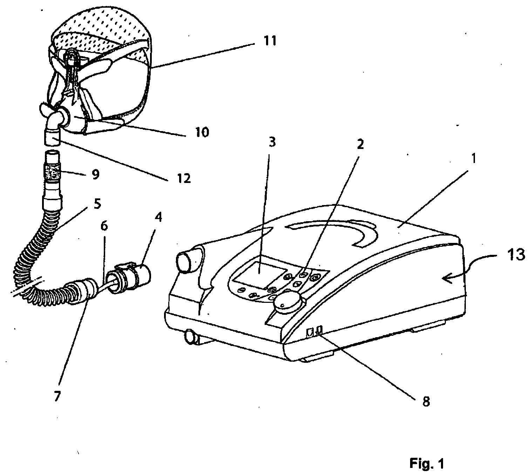

Fig. 1 zeigt den grundsätzlichen Aufbau einer Vorrichtung zur Beatmung. Im Bereich eines Gerätegehäuses (1) des Beatmungsgerätes (13), mit einer Atemgasquelle im Geräteinnenraum, sind ein Bedienelement (2) und/oder ein Bedien-und Informationssystem (3), bestehend aus einer Anzeige, einer optional berührungsempfindlichen Eingabeeinheit mit optional zumindest einem Bedienfeld angeordnet. Eine Steuereinheit (14) dient dazu, den, bevorzugt auch möglichen, Echtzeitbetrieb des Beatmungsgerätes zu steuern. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) zumindest eine Schnittstelle (8) auf. Ein Anfeuchter kann zudem adaptiert werden. Im Bereich einer dem Gerätegehäuse (1) abgewandten Ausdehnung des Verbindungsschlauches (5) kann ein Ausatmungselement (9) angeordnet sein. Alternativ kann ein Ausatemventil verwendet werden.

Fig. 1 zeigt darüber hinaus ein Patienten-Interface (10), das als Nasalmaske realisiert ist. Alternativ kann das Patienten-Interface als Tubus, Trachealkanüle, Nasalkissen, Mundstück oder sogenannter Strohhalm ausgebildet sein. Eine Fixierung im Bereich eines Kopfes eines Patienten kann über eine Kopfhaube (11) erfolgen. Im Bereich seiner dem Verbindungsschlauch (5) zugewandten Ausdehnung weist das Patienten-Interface (10) ein Kupplungselement (12) auf.

Das Gesamtsystem es besteht beispielhaft aus Bedienelement (2), Bedien- und Informationssystem (3) (auch als MMI ausgebildet), Verbindungsschlauch (5), Schnittstelle (8), Patienten-Interface (10), Steuereinheit (14) mit Steuermodulen (18, 20, 23, 24), Sensor-Einrichtung (15), Statistikmodul (16), Speicher (17), Drucksteuerungsmodul (18) mit Schnittstelle (18'), Druckgenerator (19) der beispielsweise als elektronisches Gebläse oder Druckgasquelle ausgeführt ist, Volumensteuerungs-Modul (20) mit Schnittstelle (20"), Akzeptanzfenster (21), Zeitkontrollmodul (22) mit Schnittstelle (22'), Triggermodul (23) mit Schnittstelle (23'), Alarmmodul (24) mit Schnittstelle (24').

Über die Schnittstelle (8) kann die Eingabe und / oder Ausgabe von Daten, wie die Beatmungsparameter, erfolgen. Die Schnittstelle kann kabelgebunden (Bsp. USB, LAN, RS232/485 oder ähnlich) oder drahtlos (Bsp. Infrarot, - Bluetooth, WLAN, etc.) realisiert sein. Bevorzugt kann auch ein Kartenschacht vorgesehen sein. Die Schnittstelle (8) kann auch als LAN-Schnittstelle oder als sonstige Schnittstelle, auch zur Anbindung an das Internet ausgeführt sein. Im Bereich eines Gerätegehäuses kann ein Sauerstoffzuschaltventil an die Vorrichtung zur Beatmung adaptiert werden. Es ist denkbar, das Atemgas zusätzlich mit Sauerstoff anzureichern, um die Patientenversorgung zu verbessern. Über die Schnittstelle (8) - beispielsweise als Kartenschacht oder USB ausgeführt - können auch therapiefremde Daten in das erfindungsgemäße Beatmungsgerät geladen werden beziehungsweise von diesem ausgeführt werden. Das erfindungsgemäße Beatmungsgerät (13) ist so ausgelegt, dass es über einen Schlauch und ein Patienteninterface mit einem Patienten verbunden werden kann, um eine Beatmung bereitzustellen. Es umfasst eine Quelle für Atemgas, die beispielsweise als ein Elektromotor mit Gebläserad ausgebildet ist, und zumindest eine Sensor-Einrichtung (15) zur Ermittlung von Druck und/oder Fluss und/oder Volumen des Atemgases, sowie eine Steuereinheit (14), die so ausgelegt ist, dass sie für jeden Atemzyklus auf der Basis eines vorbestimmten Wertes für den Patienten und/oder auf Basis von Messsignalen für die Parameter Druck und/oder Flow und/oder Volumen einen Atemgasdruck bestimmt und die Quelle für Atemgas derart reguliert, dass der Atemgasdruck erzeugt wird.

Die Steuereinheit (14) ist ferner so ausgelegt, dass sie den aktuellen Druck und/oder Flow und/oder das Volumen von Atemgas bestimmt. Der aktuelle Wert kann über das mit der Steuereinheit verbundene Bedien- und Informationssystem (3) darstellt werden. Die Steuereinheit (14) ist außerdem so ausgelegt, dass sie bezogen auf einen oder mehrere Parameter mittels eines Statistikmoduls (16) Trendveränderungen über eine Zeitdauer bestimmen kann, wobei die Trendveränderungen im Speicher (17) abgelegt werden oder auf der Anzeige angezeigt werden können.

Weiterhin kann die Steuereinheit (14) solche Parameter-Werte, die von einem Benutzer vorgegeben wurden vergleichen, beispielsweise obere und untere Druckgrenzen oder ein Zielvolumen, oder eine maximal tolerierbare Leckage, mit den aktuellen Werten und generiert eine Benutzer-Information zu Abweichungen von der Vorgabe. Die Benutzerinformation wird bevorzugt über das Bedien- und Informationssystem (3) im Bereich der Anzeige (14) graphisch visualisiert.

Das erfindungsgemäße Beatmungsgerät weist zudem optional oder ergänzend ein Drucksteuerungsmodul auf. Das Drucksteuerungsmodul (18) steuert den Druckgenerator (19) an. Das Drucksteuerungsmodul bietet eine Schnittstelle (18') mit der Daten von anderen Modulen empfangen und an andere Module gesandt werden können. Entsprechend der im Speicher (17) hinterlegten Druckwerte und/oder dem im Speicher hinterlegten Zieldruck und/oder dem im Speicher hinterlegten Druckprofil wird der Druckgenerator zumindest in der Inspirationsphase angesteuert.

Das erfindungsgemäße Beatmungsgerät weist zudem optional oder ergänzend ein Volumensteuerungs-Modul (20) auf. Das Volumensteuerungs-Modul erhält Daten des Flusssensors und/oder Drucksensors (15) und ermittelt daraus dass in der Inspirationsphase geförderte Atemgasvolumen. Das Volumensteuerungs- Modul bietet eine Schnittstelle (20') mit der Daten von anderen Modulen empfangen und an andere Module gesandt werden können. Das Volumensteuerungs-Modul vergleicht das in der Inspirationsphase geförderte Atemgasvolumen mit dem im Speicher hinterlegten Zielvolumen. In Abhängigkeit von der Erreichung des Zielvolumens generiert das Volumensteuerungs-Modul ein Triggersignal, welches den Druckgenerator direkt oder indirekt zur Umschaltung in eine Exspirationsphase veranlasst. Das Volumensteuerungs-Modul stellt ein Akzeptanzfenster (21) für das Zielvolumen bereit. Das Akzeptanzfenster erlaubt in einer Ausführung eine Abweichung von bis zu 20 Prozent vom Zielvolumen nach oben und nach unten. Das Akzeptanzfenster erlaubt in einer ähnlichen Ausführung eine Abweichung von bis zu 15 Prozent vom Zielvolumen nach oben und nach unten. Das Akzeptanzfenster erlaubt in einer weiteren Ausführung eine Abweichung von bis zu 10 Prozent vom Zielvolumen nach oben und nach unten.

Das erfindungsgemäße Beatmungsgerät weist zudem optional oder ergänzend ein Zeitkontrollmodul (22) auf. Das Zeitkontrollmodul weist einen Speicher für den Zeitstempel des Inspirationsbeginns und eine Zeiterfassung auf. Das Zeitkontrollmodul greift über seine Schnittstelle (22') auf die Status Informationen zu in welcher Phase sich die Ventilation befindet. Das Zeitkontrollmodul erfasst in der Inspiration die vergangene Zeit und stellt diese anderen Modulen über seine Schnittstelle bereit.

Das erfindungsgemäße Beatmungsgerät weist zudem optional oder ergänzend ein Triggermodul (23) auf. Das Triggermodul ermittelt, ob der exspiratorische Trigger ausgelöst werden kann indem ermittelt wird ob das Zielvolumen erreicht wurde oder ob die maximale Inspirationszeit erreicht wurde. Das Triggermodul (23) greift über seine Schnittstelle (23') auf das verabreichte Volumen zu und vergleicht dieses mit dem gespeicherten Zielvolumen. Ist das Zielvolumen erreicht wird der exspiratorische Trigger ausgelöst. Das Triggermodul greift über seine Schnittstelle auf die vergangene Zeit seit Inspirationsbeginn zu und vergleicht dieses mit der gespeicherten maximalen Inspirationszeit.

In einer Ausführungsform überwacht das Zeitkontroll- Modul (22) den zeitlichen Verlauf der Inspiration. Dieses Zeitkontroll- Modul beendet nach Ablauf einer maximalen Inspirationszeit Ti,max die Inspiration, womit das Drucksteuerungsmodul mit einem Druckübergang auf einen exspiratorischen Druck beginnt. In einer weiteren Ausführungsform löst ein Alarmmodul (24) mit dem Ende der Inspiration ein akustisches Alarmsignal dann aus, wenn das Zielvolumen nicht im Rahmen eines Akzeptanzfensters erreicht wird.

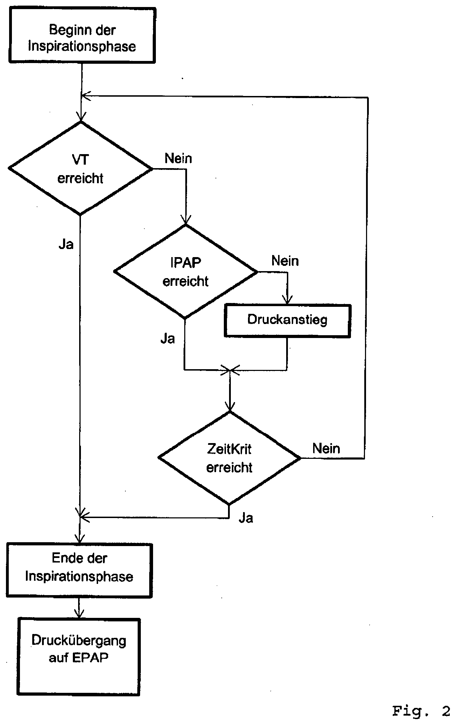

Fig. 2 veranschaulicht den gerätetechnischen Ablauf der Inspirationsphase. Die Inspirationsphase wird mandatorisch oder spontan durch einen Patienten-Trigger, der sensorisch erfasst wird, begonnen. Das Beatmungsgerät appliziert den eingestellten Druck entsprechend dem/den Vorgabewert(en), optional wird der Druck gemäß einer eingestellten Druckanstiegsrampe erhöht. Ist der eingestellte IPAP erreicht, so wird der Druck nicht weiter erhöht. Der Druck wird gehalten.

Das Beatmungsgerät (13) ermittelt über den Flow-Sensor (15) durch Integration des Flowsignals das abgegebene Volumen an Atemgas und vergleicht es mit dem eingegebenen Zielvolumen. Das abgegebene Volumen wird weiterhin überwacht und sobald das abgegebene Volumen dem eingestellten Volumen entspricht oder sich diesem im Rahmen eines Akzeptanzfensters annähert wird die Inspirationsphase beendet.

Optional wird die Zeit seit Beginn der Inspirationsphase ermittelt und mit der maximalen Inspirationszeit verglichen. Wird die maximale Inspirationszeit überschritten so wird die Inspirationsphase beendet.

Sobald die Inspirationsphase beendet ist beginnt der Druckübergang auf den EPAP.

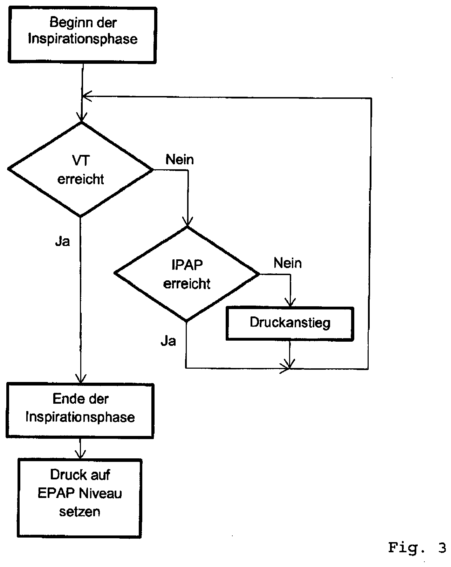

Fig. 3 veranschaulicht den gerätetechnischen Ablauf der Inspirationsphase. Die Inspirationsphase wird mandatorisch oder spontan durch einen Patienten-Trigger begonnen. Das Beatmungsgerät appliziert den eingestellten Druck, optional wird der Druck gemäß der eingestellten Druckanstiegsrampe erhöht. Ist der eingestellte IPAP erreicht, so wird der Druck nicht weiter erhöht. Der Druck wird auf einem Plateau (IPAP) gehalten.

Das Beatmungsgerät ermittelt über den Flow-Sensor durch Integration das abgegebene Volumen an Atemgas und vergleicht es mit dem eingegebenen Zielvolumen. Das abgegebene Volumen wird weiterhin überwacht und sobald das abgegebene Volumen dem eingestellten Volumen entspricht oder sich diesem annähert wird die Inspirationsphase beendet.

Optional wird die Zeit seit Beginn der Inspirationsphase ermittelt und mit der maximalen Inspirationszeit verglichen. Wird die maximale Inspirationszeit überschritten so wird die Inspirationsphase beendet.

Mit Beendigung der Inspirationsphase wird das Druckniveau auf den EPAP-Wert abgesenkt.

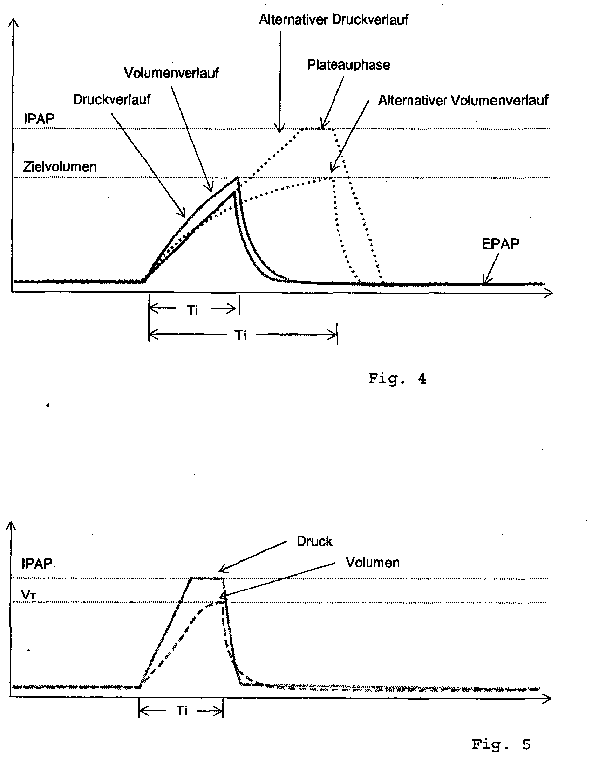

Fig. 4 zeigt den Druck- und Volumenverlauf einer erfindungsgemäßen Ventilation mit möglichen Verläufen, wobei der eingestellte maximale Druck abhängig vom Volumenverlauf erreicht wird oder nicht. Die Inspirationsphase wird mandatorisch oder spontan durch einen Patienten-Trigger ausgelöst. Das Beatmungsgerät appliziert den eingestellten Druck, optional wird der Druck gemäß der eingestellten Druckanstiegsrampe erhöht. Ist der eingestellte IPAP erreicht, so wird der Druck entlang einer Plateauphase konstant gehalten, bis das Volumen appliziert ist. Erfindungsgemäß ist auch daran gedacht, Werte für Zielvolumen und Druckverlauf so zu wählen, dass das Zielvolumen erwartungsgemäß vor Erreichung des maximalen Druckes abgegeben ist. In diesem Fall wirkt die Druckdifferenz zum maximalen Druck als eine Druckreserve, die z.B. bei einer Verschlechterung des Lungenstatus wirksam werden kann. Das Beatmungsgerät ermittelt über den Flow-Sensor durch Integration das abgegebene Volumen an Atemgas und vergleicht es mit dem eingegebenen Zielvolumen. Das abgegebene Volumen wird weiterhin überwacht und sobald das abgegebene Volumen dem eingestellten Ziel-Volumen im Bereich eines
Akzeptanzfensters entspricht wird die Inspirationsphase beendet. Sobald die Inspirationsphase beendet ist wird das Druckniveau auf den EPAP-Wert gesetzt.

Der Zusammenhang der physikalischen Größen wird zum besseren Verständnis der Erfindung im Folgenden dargestellt.

Druck (p)
Flow (F) = Ableitung des Volumens nach der Zeit
Volumen (V)
Resistance (R)
Compliance (C)
Elastance (E)

$$R = \Delta p / F$$

$$C = \Delta V / \Delta p$$

$$\tau = R \times C$$

$$E = 1 / C \text{ bzw. } C = 1 / E$$

$$pelast = V \times E \text{ ( oder } V / C)$$

$$presist = F \times R$$

Zu überwindender Druck in der Inspiration

$$pgesamt = pelast + presist$$

Dieses pgesamt wird von dem Patienten selber (prespirator = 0) oder vom Beatmungsgerät (ppatient = 0) oder von beiden aufgebracht

$$pgesamt = prespirator + ppatient$$

$$Pgesamt = prespirator + ppatient = pelast + presist = V \times E + F \times R$$

Je höher E (oder je niedriger C) und je höher R desto höher ist der Druck für die Bewegung eines bestimmten Volumens.

$$\text{Atemarbeit ist } W = p \times V$$

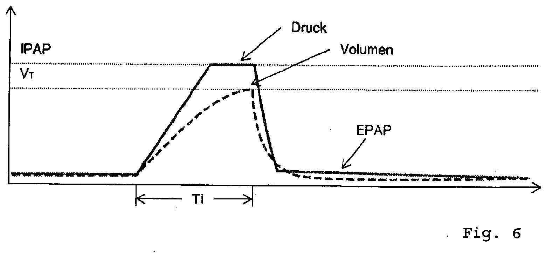

Fig. 5 und Fig. 6 zeigen zwei Druck- und Volumenverläufe einer erfindungsgemäßen Ventilation mit unterschiedlichen Druckanstiegsrampen, sowie dem Tidalvolumen (VT), das als Abschaltkriterium dienen kann, und dem inspiratorischen Druckniveau (IPAP). Es wird gezeigt, dass bei gleichbleibenden Patienten- und Gerätesetting, die Inspirationszeit bis das Zielvolumen erreicht wird durch die Wahl der Rampe beeinflusst wird. Eine flache Rampe bewirkt eine Verlängerung des Ti eine steielere Rampe eine Verkürzung. Die resultierende Inspirationszeit kann somit durch die Wahl der Druckanstiegsrampe beeinflusst werden.

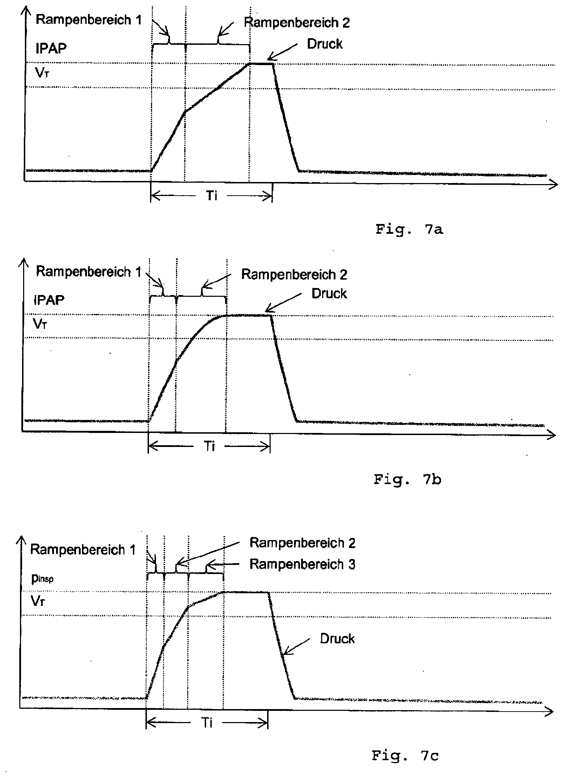

Fig. 7 zeigt mehrere erfindungsgemäße alternative Druckanstiegsrampen als Drucksollkurve, die über Rampenbereiche mit verschiedener Steigung verlaufen. Beispielsweise linear, stetig steigend, rampenförmig, mit Rampenbereichen mit weichen übergängen also mathematische stetig differenzierbar, oder mit abruptem Übergang zwischen linearen Steigungen, also mathematische nicht stetig differenzierbar.

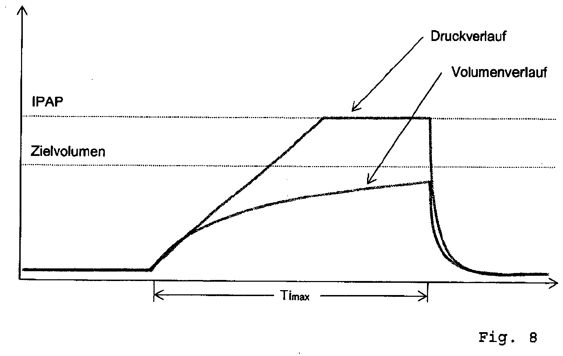

Fig. 8 zeigt den Druck- und Volumenverlauf einer erfindungsgemäßen Ventilation bei der das Ziel-Volumen bis zum Ablauf von Ti,max nicht erreicht wird. Wird, wie in Fig. 8 gezeigt, das Volumen nicht innerhalb einer bestimmten Zeit - also auch nicht während der Plateauphase - erreicht, wird die Inspiration mit Ablauf von Ti,max abgebrochen.

In einer Ausführungsform wird auch ein Alarm ($VT_{low}$) generiert, wenn nach Ablauf der Zeit Ti,max das eingestellte Volumen nicht erreicht ist. Die Zeit Ti,max beträgt in einer Ausführungsform 4 sec. Der $VT_{low}$ Alarm wird in einer Ausführung generiert, wenn in einem Atemzug nicht das Zielvolumen erreicht wird. Der $VT_{low}$ Alarm wird einer anderen Ausführung erst dann generiert, wenn in mehreren aufeinander folgenden Atemzügen nicht das Zielvolumen erreicht wird.

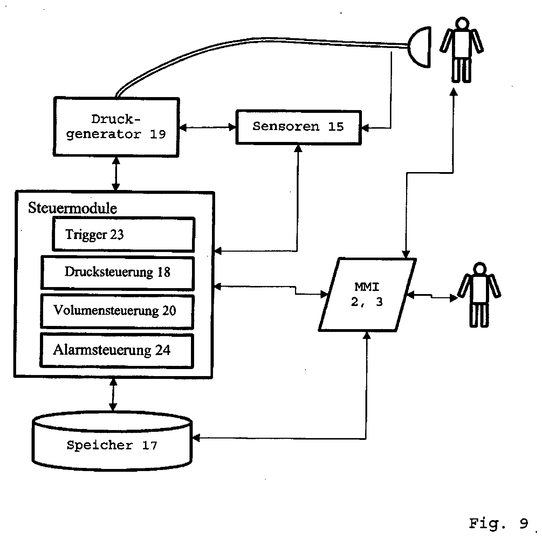

Fig. 9 zeigt das Gesamtsystem es besteht beispielhaft aus Bedienelement (2), Bedien- und Informationssystem (3) (hier als MMI ausgebildet), Verbindungsschlauch (5), Schnittstelle (8), Patienten-Interface (10), Steuereinheit (14), Sensor-Einrichtung (15), Statistikmodul (16), Speicher (17), Drucksteuerungsmodul (18) mit Schnittstelle (18'), Druckgenerator (19), Volumensteuerungs-Modul (20) mit Schnittstelle (20'), Akzeptanzfenster (21), Zeitkontrollmodul (22) mit Schnittstelle (22'), Triggermodul (23) mit Schnittstelle (23'), Alarmmodul (24) mit Schnittstelle (24').

Der Druckgenerator (19) erzeugt beispielsweise abhängig der Vorgaben der Steuermodule der Steuereinheit (14) einen Druck behafteten Fluss eines atembaren Gases. Die Sensoren (15) nehmen Messwerte auf, verarbeiten diese und stellen sie den Modulen zur Verfügung.

Das Drucksteuerungs-Modul ermittelt und/oder überwacht und/oder erhöht beispielsweise den Druck bis maximal der vorgegebene inspiratorische Druck (IPAP) erreicht ist. Der Verlauf des insp. Druckes ($P_{insp}$) ist abhängig von der vorgegebenen Rampe. Die Rampe ist im Speicher abgelegt oder algorithmisch berechnet aufgrund von Anwendervorgaben. Das Volumensteuerungs-Modul ermittelt und/oder überwacht beispielsweise permanent das abgegebene Volumen und vergleicht es mit der Vorgabe. Beendet die Inspirationsphase, wenn das Zielvolumen erreicht ist. Das Trigger-Modul löst spontan und/oder zeitgesteuert die Inspiration aus.

Das Alarmsteuerungs-Modul ermittelt und/oder überwacht beispielsweise den zeitlichen Verlauf der Inspiration und beendet nach einer vorgegebenen Zeit die Inspiration.

Der Speicher (17) speichert beispielsweise Daten die für die Ventilation relevant sind unter anderem Parameter, Messwerte, Compliance

Das MMI (Mensch-Maschine-Interface) empfängt und/oder verarbeitet beispielsweise Benutzereingaben und zeigt Informationen, Parameter, Messwerte und Alarme.

Der Patient atmet ein atembares Gas, dass durch den Druckgenerator mit einem Druck beaufschlagt wird. Sensoren liefern Messwerte über physikalischen Größen des atembaren Gases und stellen diese dem Druckgenerator und den Steuermodulen zur Verfügung. Die Steuermodule generieren unter Berücksichtigung der Vorgaben des MMI, das die Benutzereingaben und die Anzeige übernimmt, ein Druckprofil, welches es an den Druckgenerator weiter gegeben wird. Die Steuermodule sind ein Triggermodul, dieses hat zu Aufgabe mit Hilfe von Sensoren oder anderen Algorithmen die Beatmungsphasen zu steuern, ein Drucksteuerungsmodul, das ein Druckprofil erzeugt, ein Volumensteuerungsmodul, dieses regelt den Druckgenerator, beispielsweise wenn eine Volumenüberwachung eingestellt ist. Ebenfalls beinhalten die Steuermodule optional eine Alarmsteuerung diese wertet unter anderem die Sensordaten, die durch das MMI gewonnenen Daten, die im Speicher (17) hinterlegten Daten, sowie die Zustände der anderen Steuermodule aus und reagiert auf die Ergebnisse. Die von den Steuermodulen gewonnenen Daten werden im Speicher hinterlegt oder von den Steuermodulen für ihre Verarbeitung geladen.

## Patentansprüche

1. Beatmungsgerät (13) mit einem Druckgenerator (19) zur Förderung von Atemgas unter Inspirationsdruck und Exspirationsdruck zu einem Patienten Interface (10), mit zumindest einem Flow- und/oder Drucksensor (15), einer Eingabeeinheit (2, 3) zumindest zur Vorgabe eines Zielvolumens für eine Inspirationsphase sowie zumindest einem Speicher (17) zur abrufbaren Speicherung von Zielvolumen und von Druckwerten und einer Steuereinheit (14), die zumindest ein Steuermodul (18, 20, 23, 24) aufweist, **dadurch gekennzeichnet, dass** der Druckgenerator (19) während der Inspirationsphase das Atemgas unter Druck fördert und das Volumensteuerungs-Modul (20) der Steuereinheit (14) das geförderte Atemgasvolumen ermittelt und mit dem Zielvolumen vergleicht und es den Druckgenerator (19) in Abhängigkeit von der Erreichung des Zielvolumens zur Umschaltung in eine Exspirationsphase veranlasst.

**2.** Beatmungsgerät nach Anspruch 1 **dadurch gekennzeichnet, dass** das Volumensteuerungs-Modul (20) ein Akzeptanzfenster (21) für das Zielvolumen bereitstellt.

**3.** Beatmungsgerät nach Anspruch 2 **dadurch gekennzeichnet, dass** das Akzeptanzfenster (21) eine Abweichung von bis zu 20 Prozent vom Zielvolumen umfasst.

**4.** Beatmungsgerät nach zumindest einem der vorhergehenden Ansprüche **dadurch gekennzeichnet, dass** das Volumensteuerungs-Modul (20) Daten des Flusssensors und/oder Drucksensors (15) erhält und daraus das in der Inspirationsphase geförderte Atemgasvolumen ermittelt, wobei das Volumensteuerungs-Modul eine Schnittstelle (20') aufweist über die Daten mit anderen Modulen (18, 23, 24) ausgetauscht werden und wobei das Volumensteuerungs-Modul das in der Inspirationsphase geförderte Atemgasvolumen mit dem im Speicher (17) hinterlegten Zielvolumen vergleicht und in Abhängigkeit von der Erreichung des Zielvolumens ein Triggersignal generiert, welches den Druckgenerator (19) direkt oder indirekt (über das Drucksteuerungsmodul (18)) zur Umschaltung in eine Exspirationsphase veranlasst.

**5.** Beatmungsgerät nach zumindest einem der vorhergehenden Ansprüche **dadurch gekennzeichnet, dass** die Steuereinheit (14) ein Drucksteuerungsmodul (18) aufweist, welches den Druckgenerator (19) ansteuert und welches eine Schnittstelle (18') aufweist, über die Daten mit anderen Modulen (20, 23, 24) ausgetauscht werden, wobei das Drucksteuerungsmodul (18) den Druckgenerator zumindest in der Inspirationsphase entsprechend der im Speicher (17) hinterlegten Druckwerte und/oder dem im Speicher hinterlegten Zieldruck und/oder dem im Speicher hinterlegten Druckprofil angesteuert.

**6.** Beatmungsgerät nach zumindest einem der vorhergehenden Ansprüche **dadurch gekennzeichnet, dass** die Steuereinheit (14) ein Zeitkontrollmodul (22) aufweist, welches in dem Speicher (17) einen Zeitstempel des Inspirationsbeginns und eine Zeiterfassung der Inspiration aufzeichnet, wobei das Zeitkontrollmodul (22) über seine Schnittstelle (22'), über die Daten mit anderen Modulen ausgetauscht werden, auf die Status Information, in welcher Phase sich die Ventilation befindet, zugreift und wobei das Zeitkontrollmodul die in der Inspiration verstrichene Zeit erfasst und diese anderen Modulen über seine Schnittstelle bereitstellt.

**7.** Beatmungsgerät nach zumindest einem der vorhergehenden Ansprüche **dadurch gekennzeichnet, dass** die Steuereinheit (14) ein Triggermodul (23) aufweist, welches ermittelt, ob der exspiratorische Trigger ausgelöst werden kann, indem ermittelt wird, ob das Zielvolumen erreicht wurde oder ob die maximale Inspirationszeit erreicht wurde, wobei das Triggermodul (23) über seine Schnittstelle (23') das verabreichte Volumen an Atemgas abgreift und dieses mit dem gespeicherten Zielvolumen vergleicht und wobei das Triggermodul über seine Schnittstelle die Zeit seit Inspirationsbeginn abgreift und diese mit der gespeicherten maximalen Inspirationszeit vergleicht und wobei das Triggermodul den exspiratorischen Trigger ausgelöst, wenn das Zielvolumen erreicht ist und/oder die maximalen Inspirationszeit erreicht ist.

**8.** Beatmungsgerät nach zumindest einem der vorhergehenden Ansprüche **dadurch gekennzeichnet, dass** die Steuereinheit (14) ein Zeitkontroll-Modul (22) aufweist, welches den zeitlichen Verlauf der Inspiration überwacht und aufzeichnet, wobei das Zeitkontroll-Modul die Inspiration beendet wenn die maximale Inspirationszeit Ti,max abgelaufen ist, woraufhin das Drucksteuerungsmodul mit einem Druckübergang auf einen exspiratorischen Druck beginnt.

**9.** Beatmungsgerät nach zumindest einem der vorhergehenden Ansprüche **dadurch gekennzeichnet, dass** die Steuereinheit (14) ein Alarmmodul (24) aufweist, welches mit dem Ende der Inspiration ein akustisches Alarmsignal generiert, wenn das Zielvolumen nicht im Rahmen des Akzeptanzfensters erreicht wird.

**10.** Beatmungsgerät nach zumindest einem der vorhergehenden Ansprüche **dadurch gekennzeichnet, dass** der Druckgenerator (19) während der Inspirationsphase das Atemgas unter dem vorgegebenen Druck oder entsprechend dem vorgegebenen Druckprofil fördert.

**11.** Beatmungsgerät nach zumindest einem der vorhergehenden Ansprüche **dadurch gekennzeichnet, dass** der Druckgenerator alternative Druckanstiegsrampen als Drucksollkurve vorgibt und/oder ausgewählt aus Rampen mit verschiedener Steigung oder linear oder stetig steigend oder rampenförmig oder mit weichen Übergängen.

**12.** Beatmungsgerät nach zumindest einem der vorhergehenden Ansprüche **dadurch gekennzeichnet, dass** über die Eingabeeinheit (2, 3) die Vorgabe eines Zielvolumens und zumindest eines Druckwertes oder einer Druckanstiegsrampe für eine Inspirationsphase erfolgt.

**13.** Beatmungsgerät nach zumindest einem der vorhergehenden Ansprüche **dadurch gekennzeichnet, dass** von der Inspiration in die Exspiration geschaltet wird, wenn nach Ablauf einer definierbaren Zeitspanne das Zielvolumen nicht erreicht wurde.

**14.** Beatmungsgerät nach zumindest einem der vorhergehenden Ansprüche **dadurch gekennzeichnet, dass** nach Ablauf der maximalen Inspirationszeit ein akustischer und/oder optischer Alarm ausgelöst wird.

**Claims**

**1.** A ventilator (13) having a pressure generator (19) for conveying respiratory gas under inspiration pressure and expiration pressure to a patient interface (10) with at least one flow and/or pressure sensor (15), an input unit (2, 3) at least for specifying a target volume for an inspiration phase, and at least one memory (17) for retrievably saving target volumes and pressure values, and a control unit (14) that at least has a control module (18, 20, 23, 24), **characterized in that** the pressure generator (19) conveys the respiratory gas under pressure during the inspiration phase, and the volume control module (20) of the control unit (14) determines the delivered respiratory gas volume and compares it with the target volume, and causes the pressure generator (19) to switch to an expiration phase as a function of reaching the target volume.

**2.** The ventilator according to claim 1, **characterized in that** the volume control module (20) provides an acceptance window (21) for the target volume.

**3.** The ventilator according to claim 2, **characterized in that** the acceptance window (21) comprises a deviation of up to 20% from the target volume.

**4.** The ventilator according to at least one of the preceding claims, **characterized in that** the volume control module (20) receives data from the flow sensor and/or pressure sensor (15) and determines therefrom the respiratory gas volume delivered in the inspiration phase, wherein the volume control module has an interface (20’) by means of which data are exchanged with other modules (18, 23, 24), and wherein the volume control module compares the respiratory gas volume delivered in the inspiration phase with the target volume saved in the memory (17) and, as a function of reaching the target volume, generates a trigger signal that causes the pressure generator (19) directly or indirectly (via the pressure control module (18)) to switch to an expiration phase.

**5.** The ventilator according to at least one of the preceding claims, **characterized in that** the control unit (14) has a pressure control module (18) that controls the pressure generator (19), and that has an interface (18’) by means of which the data are exchanged with other modules (20, 23, 24), wherein the pressure control module (18) controls the pressure generator, at least in the inspiration phase, corresponding to the pressure values saved in the memory (17), and/or the target pressure saved in the memory, and/or the pressure profile saved in the memory.

**6.** The ventilator according to at least one of the preceding claims, **characterized in that** the control unit (14) has a time monitoring module (22) that records in the memory (17) a timestamp of the start of inspiration and detected inspiration time, wherein the time monitoring module (22) accesses the status information on the existing phase of ventilation via its interface (22’), by means of which data are exchanged with other modules, and wherein the time monitoring module detects the time passed during inspiration and provides this to other modules via its interface.

**7.** The ventilator according to at least one of the preceding claims, **characterized in that** the control unit (14) has a trigger module (23) that determines whether the expiratory trigger can be triggered by determining if the target volume was reached, or if the maximum inspiration time was reached, wherein the trigger module (23) accesses the administered volume of respiratory gas via its interface (23’) and compares it with the saved target volume, and wherein the trigger module accesses the time since the start of inspiration via its interface and compares it with the saved maximum inspiration time, and wherein the trigger module triggers the expiratory trigger when the target volume is reached, and/or the maximum inspiration time is reached.

**8.** The ventilator according to at least one of the preceding claims, **characterized in that** the control unit (14) has a time monitoring module (22) that monitors and records the duration of inspiration, wherein the time monitoring module terminates inspiration when the maximum inspiration time Ti,max has passed, then the pressure control module starts a pressure transition to an expiratory pressure.

**9.** The ventilator according to at least one of the preceding claims, **characterized in that** the control unit (14) has an alarm module (24) that generates an acoustic alarm signal upon the end of inspiration when the target volume is not reached within the acceptance window.

**10.** The ventilator according to at least one of the preceding claims, **characterized in that** the pressure generator (19) delivers the respiratory gas during the inspiration phase under the given pressure, or corresponding to the given pressure profile.

**11.** The ventilator according to at least one of the preceding claims, **characterized in that** the pressure generator specifies and/or selects alternative pressure rise ramps as a pressure target curve from ramps with a different slope, or linear or continuously rising, or ramp-like, or with soft transitions.

**12.** The ventilator according to at least one of the preceding claims, **characterized in that** a target volume and at least one pressure value, or a pressure rise ramp for an inspiration phase is specified by the input unit (2, 3).

**13.** The ventilator according to at least one of the preceding claims, **characterized in that** inspiration switches to expiration when the target volume is not reached after the passage of a specifiable time span.

**14.** The ventilator according to a least one of the preceding claims, **characterized in that** after passage of the maximum inspiration time, an acoustic and/or visual alarm is triggered.

## Revendications

**1.** Respirateur (13) avec un générateur de pression (19) pour le transport de gaz respiratoire sous pression -d'inspiration et pression d'expiration vers une interface de patient (10), avec au moins un capteur de flux et/ou de pression (15), une unité d'entrée (2, 3) au moins pour la prédéfinition d'un volume cible pour une phase d'inspiration ainsi qu'au moins une mémoire (17) pour la mémorisation pouvant être consultée de volumes cibles et de valeurs de pression, et une unité de commande (14) qui présente au moins un module de commande (18, 20, 23, 24), **caractérisé en ce que** le générateur de pression (19) transporte le gaz respiratoire sous pression pendant la phase d'inspiration, et le module de commande de volume (20) de l'unité de commande (14) détermine le volume de gaz respiratoire transporté et le compare au volume cible, et cela amène le générateur de pression (19) à passer à une phase d'expiration en fonction de l'atteinte du volume cible.

**2.** Respirateur selon la revendication 1, **caractérisé en ce que** le module de commande de volume (20) met à disposition une fenêtre d'acceptation (21) pour le volume cible.

**3.** Respirateur selon la revendication 2, **caractérisé en ce que** la fenêtre d'acceptation (21) comprend un écart allant jusqu'à 20 pourcent par rapport au volume cible.

**4.** Respirateur selon au moins une des revendications précédentes, **caractérisé en ce que** le module de commande de volume (20) reçoit des données du capteur de flux et/ou capteur de pression (15) et détermine à partir de celles-ci le volume de gaz respiratoire transporté dans la phase d'inspiration, dans lequel le module de commande de volume présente une interface (20') par le biais de laquelle des données sont échangées avec d'autres modules (18, 23, 24) et dans lequel le module de commande de volume compare le volume de gaz respiratoire transporté dans la phase d'inspiration au volume cible stocké dans la mémoire (17) et génère un signal déclencheur qui amène le générateur de pression (19) directement ou indirectement (par le biais du module de commande de pression (18)) à passer à une phase d'expiration en fonction de l'atteinte du volume cible.

**5.** Respirateur selon au moins une des revendications précédentes, **caractérisé en ce que** l'unité de commande (14) présente un module de commande de pression (18) qui commande le générateur de pression (19) et qui présente une interface (18') par le biais de laquelle des données sont échangées avec d'autres modules (20, 23, 24), dans lequel le module de commande de pression (18) commande le générateur de pression au moins dans la phase d'inspiration en fonction des valeurs de pression stockées dans la mémoire (17) et/ou de la pression cible stockée dans la mémoire et/ou du profil de pression stocké dans la mémoire.

**6.** Respirateur selon au moins une des revendications précédentes, **caractérisé en ce que** l'unité de commande (14) présente un module de contrôle temporel (22) qui enregistre dans la mémoire (17) une estampille temporelle du début d'inspiration et une détection temporelle de l'inspiration, dans lequel le module de contrôle temporel (22) accède par le biais de son interface (22') par le biais de laquelle des données sont échangées avec d'autres modules à l'information de statut concernant la phase dans laquelle la ventilation se trouve, et dans lequel le module de contrôle temporel détecte le temps écoulé dans l'inspiration et met à disposition celui-ci à d'autres modules par le biais de son interface.

**7.** Respirateur selon au moins une des revendications précédentes, **caractérisé en ce que** l'unité de commande (14) présente un module déclencheur (23) qui détermine si le déclencheur expiratoire peut être déclenché **en ce qu'**il est déterminé si le volume cible a été atteint ou si la durée d'inspiration maximale a été atteinte, dans lequel le module déclencheur (23) acquiert par le biais de son interface (23') le volume administré de gaz respiratoire et compare celui-ci au volume cible mémorisé, et dans lequel le module déclencheur acquiert par le biais de son interface la durée depuis le début d'inspiration et compare celle-ci à la durée d'inspiration maximale mémorisée, et dans lequel le module déclencheur déclenche le déclencheur expiratoire lorsque le volume cible est atteint et/ou la durée d'inspiration maximale est atteinte.

**8.** Respirateur selon au moins une des revendications précédentes, **caractérisé en ce que** l'unité de commande (14) présente un module de contrôle temporel (22) qui surveille et enregistre le déroulement temporel de l'inspiration, dans lequel le module de contrôle temporel termine l'inspiration lorsque la durée d'inspiration maximale Ti,max s'est écoulée, suite à quoi le module de commande de pression commence une transition de pression à une pression expiratoire.

**9.** Respirateur selon au moins une des revendications précédentes, **caractérisé en ce que** l'unité de commande (14) présente un module d'alarme (24) qui génère avec la fin de l'inspiration un signal d'alarme sonore lorsque le volume cible n'est pas atteint dans le cadre de la fenêtre d'acceptation.

**10.** Respirateur selon au moins une des revendications précédentes, **caractérisé en ce que** le générateur de pression (19) transporte pendant la phase d'inspiration le gaz respiratoire sous la pression prédéfinie ou en fonction du profil de pression prédéfini.

**11.** Respirateur selon au moins une des revendications précédentes, **caractérisé en ce que** le générateur de pression prédéfinit des rampes de hausse de pression alternatives comme courbe de consigne de pression et/ou sélectionnées parmi des rampes avec une pente différente ou à pente linéaire ou continue ou en forme de rampe ou avec des transitions douces.

**12.** Respirateur selon au moins une des revendications précédentes, **caractérisé en ce que** la prédéfinition d'un volume cible et d'au moins une valeur de pression ou d'une rampe de hausse de pression pour une phase d'inspiration s'effectue par le biais de l'unité d'entrée (2, 3).

**13.** Respirateur selon au moins une des revendications précédentes, **caractérisé en ce qu'**il est passé de l'inspiration à l'expiration lorsque le volume cible n'a pas été atteint après écoulement d'un laps de temps pouvant être défini.

**14.** Respirateur selon au moins une des revendications précédentes, **caractérisé en ce qu'**une alarme sonore et/ou visuelle est déclenchée après écoulement de la durée d'inspiration maximale.

11
10
12
9
5
6
4
7
3
2
1
13
8

Fig. 1

Beginn der Inspirationsphase
VT erreicht
Nein
Ja
IPAP erreicht
Nein
Druckanstieg
Ja
ZeitKrit erreicht
Nein
Ja
Ende der Inspirationsphase
Druckübergang auf EPAP

Fig. 2

Beginn der
Inspirationsphase
VT
erreicht
Nein
Ja
IPAP
erreicht
Nein
Druckanstieg
Ja
Ende der
Inspirationsphase
Druck auf
EPAP Niveau
setzen

Fig. 3

Alternativer Druckverlauf
Plateauphase
Volumenverlauf
Druckverlauf
Alternativer Volumenverlauf
IPAP
Zielvolumen
EPAP
Ti
Ti

Fig. 4

Druck
Volumen
IPAP
$V_T$
Ti

Fig. 5

Druck
IPAP
Volumen
$V_T$
EPAP
Ti

Fig. 6

Rampenbereich 1
Rampenbereich 2
IPAP
Druck
VT
Ti

Fig. 7a

Rampenbereich 1
Rampenbereich 2
Druck
IPAP
VT
Ti

Fig. 7b

Rampenbereich 1
Rampenbereich 2
Rampenbereich 3
pinsp
VT
Druck
Ti

Fig. 7c

Druckverlauf
Volumenverlauf
IPAP
Zielvolumen
Timax

Fig. 8

Druck-
generator 19
Sensoren 15
Steuermodule
Trigger 23
Drucksteuerung 18
Volumensteuerung 20
Alarmsteuerung 24
MMI
2, 3
Speicher 17

Fig. 9

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2005075013 A1
- US 20140373845 A1
- WO 2013182944 A1
- WO 2014199264 A1